(19) Europäisches Patentamt
European Patent Office
Office européen des brevets

(11) Publication number: **0 053 015**
**A1**

(12) **EUROPEAN PATENT APPLICATION**

(21) Application number: 81305485.5

(22) Date of filing: 20.11.81

(51) Int. Cl.³: **C 07 C 149/42**
C 07 C 148/00, A 61 K 31/135

(30) Priority: 21.11.80 JP 164862/80
29.06.81 JP 101454/81

(43) Date of publication of application: 02.06.82 Bulletin 82/22

(84) Designated Contracting States: CH DE FR GB IT LI

(71) Applicant: Takeda Chemical Industries, Ltd.
27, Doshomachi 2-Chome
Higashi-ku Osaka, 541(JP)

(72) Inventor: Meguro, Kanji
24-311, 1 Kotobuki-cho
Nishinomiya Hyogo 662(JP)

(72) Inventor: Matsuo, Takao
1-74, Shimotsubayashi-rokutanda
Nishikyo-ku Kyoto 615(JP)

(74) Representative: Laredo, Jack Joseph et al,
Elkington and Fife High Holborn House 52/54 High Holborn
London, WC1V 6SH(GB)

(54) **1-Phenylthio-2-aminopropane derivatives, their production and use.**

(57) Novel 1-phenylthio-2-aminopropane derivatives, inclusive of salts thereof, of the formula:

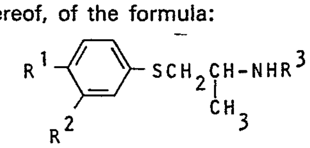

wherein $R^1$ is halogen, $R^2$ is hydrogen or halogen and $R^3$ is methyl, ethyl or hydroxyethyl, have antiobesity and antidiabetic actions.

EP 0 053 015 A1

Croydon Printing Company Ltd.

# 1-PHENYLTHIO-2-AMINOPROPANE DERIVATIVES, THEIR PRODUCTION AND USE

## BACKGROUND OF THE INVENTION

### 1. Field of the Invention

This invention relates to novel 1-phenylthio-2-aminopropane derivatives having excellent pharmacological actions.

### 2. Description of the Prior Art

Belgian Patent No. 653,101 referes to 2-phenylthioethylamine derivatives including 1-phenylthio-2-methylaminopropane as having hypertensive, hypotensive and antiepileptic properties.

South African Patent No. 67/3108 describes 1-(4-chlorophenylthio)-2-aminopropane as having an antidepressive activity.

## SUMMARY OF THE INVENTION

The present inventors have succeeded in synthesizing novel 1-phenylthio-2-aminopropane derivatives of the formula:

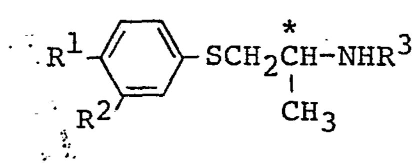

(I)

wherein $R^1$ is halogen; $R^2$ is hydrogen or halogen; and $R^3$ is methyl, ethyl or hydroxyethyl, and have found that these compounds and acid addition salts thereof have outstanding antiobesity and antidiabetic actions.

0053015



Thus, the present invention provides the novel 1-phenylthio-2-aminopropane derivatives (I) and acid addition salts thereof, which have excellent pharmacological actions. This invention also provides the use of these compounds as medicaments. This invention further provides industrially feasible processes for producing these compounds.

DESCRIPTION OF THE PREFERRED EMBODIMENTS

Referring to the above-mentioned formula (I), the halogen for $R^1$ and $R^2$ includes fluorine, chlorine and bromine. When $R^2$ is halogen, this halogen and the halogen for $R^1$ may be the same or different.

Among the compounds (I), preferred embodiments are those wherein $R^3$ is methyl or/and $R^1$ is chlorine.

A preferable specific embodiment in the present invention is 1-(4-chlorophenylthio)-2-methylaminopropane and its physiologically acceptable salts.

The compound (I) of this invention can be produced, for example, according to the following reaction scheme:

Process I

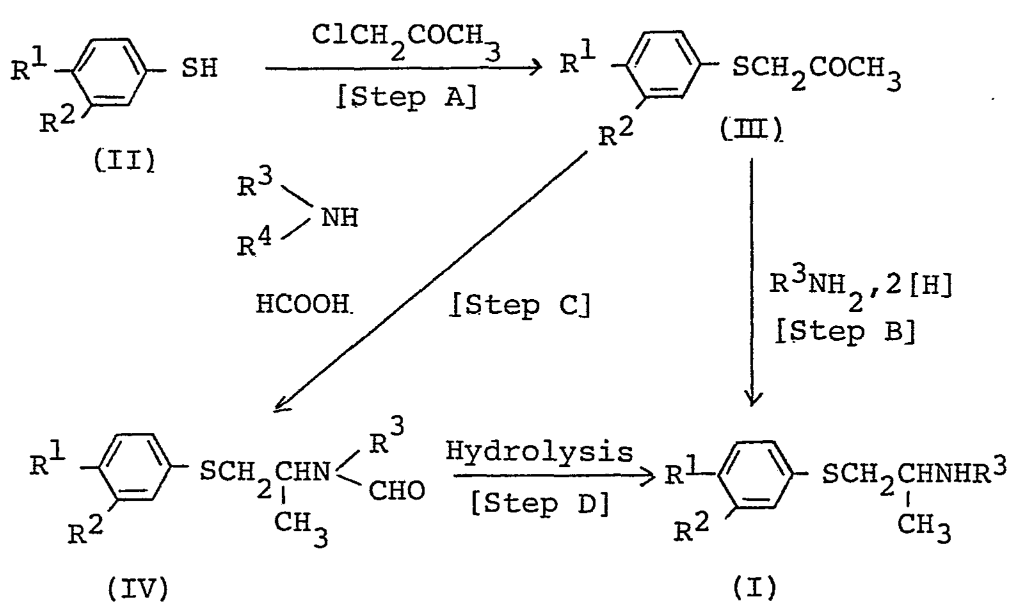

Process II

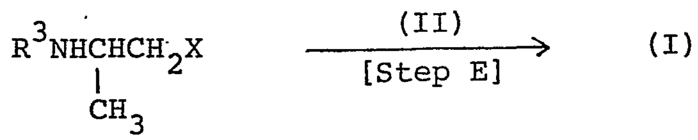


(V)

Process III

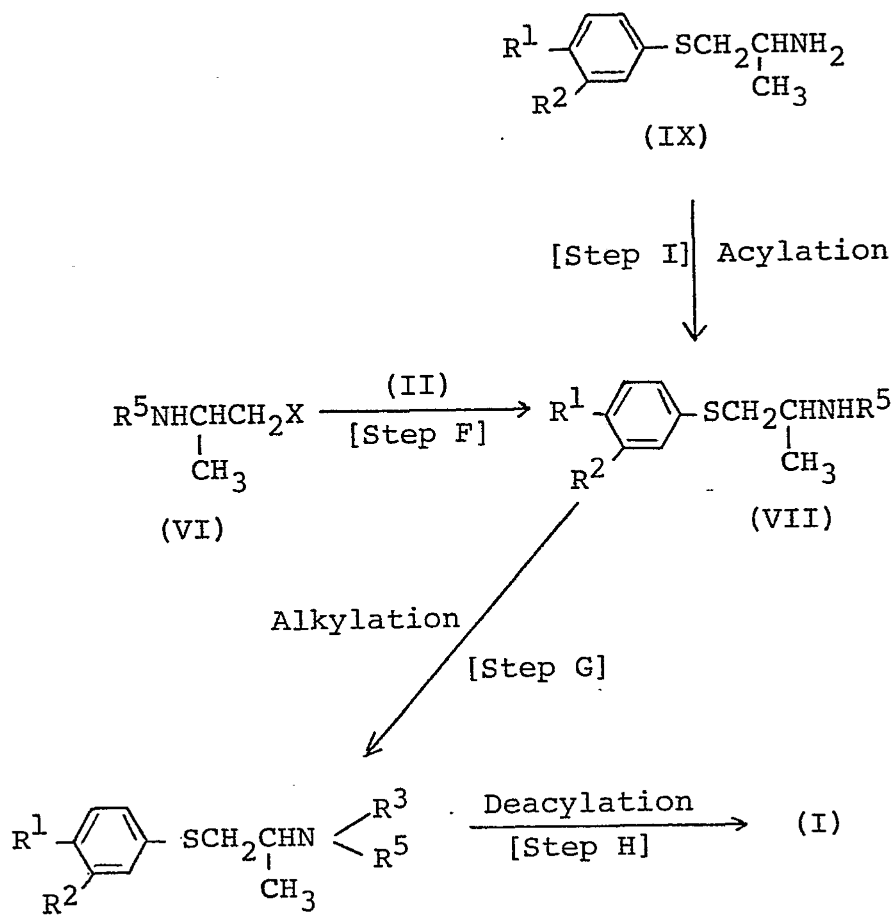


(IX) (VI) (VII) (VIII)

wherein $R^1$, $R^2$ and $R^3$ are as defined above, $R^4$ is hydrogen or formyl, $R^5$ is acyl and X is a leaving group.

In [Step A] of Process I the thiophenol derivative (II) is reacted with chloroacetone. The reaction can be conducted in a manner known *per se* at 0 to 20°C in a solvent such as methanol or ethanol in the presence of an aqueous

solution of sodium hydroxide, potassium hydroxide or the like. The resulting compound (III) can readily be collected in any purified form by distillation or recrystallization.

In [Step B], the compound (III) and the amine of the formula $R^3NH_2$ are subjected to condensation under reductive conditions to synthesize the compound (I). This reductive amination is preferably performed particularly by use of lithium or sodium cyanoborohydride ($LiBH_3CN$ or $NaBH_3CN$) and usually carried out using, per mole of the compound (III), 2 to 20 moles of $R^3NH_2$ in the presence of 1 to 3 moles of an acid such as hydrochloric acid, sulfuric acid or acetic acid in a solvent such as methanol or ethanol. The acid may be added to the reaction system in the form of a salt with $R^3NH_2$. It is usually preferred that the amount of $LiBH_3CN$ or $NaBH_3CN$ be 0.5 to 2 moles per mole of (III) and the reaction temperature be in the range of from room temperature to the boiling point of the solvent. In the reductive amination, an imine of the formula:

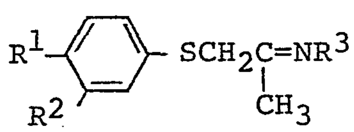

(III')

wherein $R^1$, $R^2$ and $R^3$ are as defined above, is first formed and the imine (III') is then reduced to form the desired compound (I). Therefore, it is also possible to first synthesize the imine (III') from the compound (III) and $R^3NH_2$ and then reduce it. In such cases, $NaBH_4$, $LiAlH_4$ and the like can be used as a reducing agent, in addition to the aforementioned ones.

In [Step C], the compound (III) and a primary amine or its N-formyl derivative are reacted in the presence of formic acid. This procedure utilizes the so-called Leuckart reaction which is known <u>per se</u>. The reaction may be performed by heating 1 to 20 moles, preferably 5 to 10 moles of

a primary amine or its N-formyl derivative and 1 to 10 moles of formic acid together with one mole of the compound (III) at 120 to 180°C, preferably at 130 to 170°C in the absence of a solvent. The resulting compound (IV) is subjected, after purification or directly without purification, to hydrolysis in [Step D] to synthesize the desired product (I). The hydrolysis is preferably carried out by heating with a mineral acid such as hydrochloric acid or sulfuric acid.

[Step E] of Process II is carried out by reacting a compound (V) with a thiophenol derivative (II). The leaving group X of the compound (V) is preferably a halogen which may be any of chlorine, bromine and iodine. It is generally preferred to perform this reaction in a suitable solvent in the presence of a base. As the base, for example, sodium hydroxide, potassium hydroxide, sodium methoxide, sodium ethoxide, etc. can be used conveniently. Any solvent which does not interfere with the reaction may be used, and the solvent may be suitably selected, for example, from methanol, ethanol, tetrahydrofuran, dimethoxyethane, dioxane, dimethylformamide and their aqueous solution. While the reaction proceeds at 0 to 30°C, it may be carried out under heating to the boiling point in order to accelerate the reaction. While the compounds (II) and (V) may be used in equimolar amounts, either may be present in a slight excess. The amount of the base used is 1 to 1.2 moles per mole of the compound (II), but when the compound (V) is used in the form of an acid addition salt (e.g., hydrochloride, hydrobromide, hydroiodide), an additional amount of the base should be added to neutralize the acid. When X is chlorine or bromine which is relatively less reactive, the reaction may be carried out in the presence of 0.1 to 1 mole of sodium iodide or potassium iodide per mole of the compound (V) in order to accelerate the reaction.

Regarding Process III, the acyl for $R^5$ is preferably

one shown by the formula:

$$-COR^{5'}$$

wherein $R^{5'}$ is lower alkyl, aryl, aralkyl or a group of $-OR^{5''}$ [$R^{5''}$ is lower alkyl, aryl or aralkyl]. The lower alkyl represented by $R^{5'}$ or $R^{5''}$ includes those containing 1-4 carbon atoms, such as methyl, ethyl, propyl, isopropyl, n-butyl, sec-butyl and tert-butyl. The aryl for $R^{5'}$ or $R^{5''}$ is preferably phenyl, and the aralkyl includes phenyl-lower($C_{1-2}$)-alkyl containing 7 to 8 carbon atoms, such as benzyl, phenethyl, and α-methylbenzyl. When $R^{5'}$ or $R^{5''}$ contains a benzene ring, it may have at an optional position or positions on the benzene ring such substituents as methyl, ethyl, methoxy, chlorine, bromine and nitro.

In [Step I] of Process III, a 2-amino-1-phenylthio-propane derivative (IX) is acylated by use of an acid halide or anhydride corresponding to the acyl to obtain the compound (VII). It is generally advantageous to conduct this acylation in a suitable solvent in the presence of a base. As the base, use is made of, for example, sodium hydrogencarbonate, potassium hydrogencarbonate, sodium carbonate, potassium carbonate, sodium hydroxide, potassium hydroxide, triethylamine, pyridine or the like. The solvent may be, for example, chloroform, dichloromethane, tetrahydrofuran, dioxane or dimethoxyethane. While the reaction proceeds smoothly at -20 to 30°C, it may be conducted under appropriate heating for accelerating the reaction.

A compound (VII) wherein $R^5$ is a group of $-COOR^{5''}$ ($R^{5''}$ is as defined above) may be produced also by [Step F] wherein a thiophenol derivative (II) is reacted with a compound (VI). Preferred examples of the leaving group X of the compound (VI) include, in addition to those exemplified above in connection with the compound (V), alkyl- or aryl-sulfonyloxy groups such as methanesulfonyloxy, benzenesulfonyloxy and p-toluenesulfonyloxy. The reaction between the compounds (II) and (VI) can be carried out

under the same conditions as in the aforesaid [Step E].

In the alkylation of a compound (VII) in [Step G], the compound (VII) is first reacted with a suitable base in an anhydrous solvent to give an anion, which is then reacted with a conventional alkylating agent. The base to be used is suitably selected from sodium hydride, sodium amide, sodium methoxide, sodium ethoxide, potassium t-butoxide and the like, and the solvent includes dimethylformamide, dimethyl sulfoxide, tetrahydrofuran, dioxane, dimethoxyethane and the like. The alkylating agent may be one employed in the conventional alkylation reactions and includes, for example, halides (e.g. chloride, bromide or iodide), sulfate ester (e.g. dimethylsulfate, diethylsulfate), sulfonate ester (e.g. methanesulfonate, benzenesulfonate or toluenesulfonate), etc. of the alkyl corresponding to $R^3$. The reaction is usually carried out at 0 to 40°C, but a lower or higher temperature may be employed in order to control the reaction rate.

The resulting compound (VIII) may be subjected to deacylation to form the corresponding compound (I) [Step H]. The deacylation can readily be performed by use of an acid (e.g. hydrochloric acid, hydrobromic acid, sulfuric acid) or an alkali (e.g. sodium hydroxide, potassium hydroxide, barium hydroxide), as well as, when $R^5$ is a group of $-COOR^{5''}$ ($R^{5''}$ is aralkyl or t-butyl), by the deprotection reaction which is known per se in peptide chemistry. Thus, these groups may be eliminated by reaction with hydrogen bromide-acetic acid, and p-methoxybenzyl or t-butyl for $R^{5''}$ may be eliminated by use of trifluoroacetic acid or hydrogen chloride-organic solvent or hydrogen chloride-acetic acid. In the deacylation reaction in the case where $R^{5''}$ is aralkyl or t-butyl, satisfactory results may be obtained by adding anisole, mercaptoethanol, methyl ethyl sulfide or the like so as to suppress side reactions.

The thus formed desired compound (I) can be isolated in the form of a free base or acid addition salt in a

manner which is known *per se*. For example, a free base can be purified and collected by distillation, and an acid addition salt by recrystallization. The acid addition salt is preferably a physiologically acceptable one which includes, for example, inorganic acid salts such as hydrochloride, hydrobromide, sulfate and phosphate and organic salts such as acetate, maleate, fumarate, acid fumarate, succinate, tartrate, citrate and malate.

The compound (I) includes two optically active isomers wherein the absolute configurations at the carbon asterisked in the formula (I) are the S- and R-configurations, respectively. It is a matter of course that the present invention encompasses both of these isomers as well as a mixture thereof. If desired, the individual isomers may be prepared. For example, when the reaction in Process II or III is carried out using an optically active isomer (either S- or R-configuration) as the starting compound (V), (VI) or (IX), the compound (I) can be obtained as the corresponding optically active isomer. Alternatively, if the product is a mixture of these two isomers, it can be separated into the individual optical isomers and purified by the conventional optical resolution method, for example, by forming a salt with an optically active carboxylic acid (e.g., d- or ℓ-tartaric acid, d- or ℓ-malic acid, d-camphoric acid) or an optically active sulfonic acid (e.g., d-camphorsulfonic acid), separating into the individual salts of the isomers by means of recrystallization, then converting each optically active isomer into the free base (I) and further converting the free base into an appropriate acid addition salt.

Of the thus obtained optically active compounds (I), those having the S-configuration generally exhibit more favorable physiological actions than the isomers having the R-configuration.

The compounds (I) and acid addition salts thereof according to this invention exhibit, in animals, particularly

mammals (e.g. mice, rats, guinea pigs, rabbits, cats, dogs, monkeys, human beings) highly special pharmacological actions such as anorectic action, and suppressive effects on postprandial hyperglycemia, hyperinsulinemia and lipogenesis. Obesity or diabetes accompanying obesity is known to be associated with overeating, an increase in body fat, hyperinsulinemia, a decrease in glucose tolerance, etc. Therefore, the compounds of this invention are effective preventive and therapeutic agents for obesity and diabetes. Also, since the compounds of this invention have a low toxicity and do not have an excitative action on the central nervous system, that is a material side effect commonly found in anorectic agents, they can be used with safety in therapy over a prolonged period of time. In addition, as they are well absorbed by oral administration and have good stability, they can be administered orally or parenterally with safety either as they are or in a pharmaceutical form such as powders, granules, tablets, capsules, injections, etc. wherein the active compound is mixed with appropriate pharmaceutically acceptable carriers, excipients and/or diluents, for medicinal uses such as those mentioned above. The dosage depends on species, condition, age and weight of the mammals, the administration route and the like, but when the compound is orally administered to an adult human as the prophylactic and therapeutic agent for obesity or diabetes, for example, a daily dose of about 0.1 to 60 mg/kg, particularly about 0.2 to 20 mg/kg is preferred and it is desirable to divide such daily dose into several portions so as to take at about every meal time.

As compounds similar to the compounds of this invention, Belgian Patent No. 653,101 refers to 2-phenylthioethylamine derivatives of the general formula:

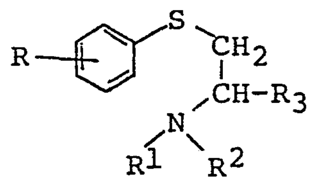

wherein R is hydrogen, hydrocarbon optionally substituted, hydroxy, alkoxy or halogen, $R_1$ and $R_2$ each are hydrogen, lower hydrocarbon, straight or branched chain, which with the N can form a heterocycle, and $R_3$ is hydrogen or lower hydrocarbon, and acid addition salts thereof, as having hypertensive, hypotensive and antiepileptic properties. But, this patent specifically described only those compounds in which the benzene ring is unsubstituted such as 1-phenylthio-2-methylaminopropane (X), and nothing is referred to therein with respect to antiobesity or antidiabetic action.

South African Patent No. 67/3108 describes the antidepressive activity of 1-(4-chlorophenylthio)-2-aminopropane of the formula:

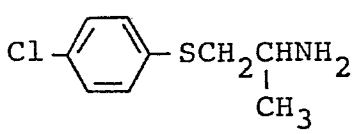 (XI)

but this patent also says nothing about antiobesity or antidiabetic action. According to the inventors' study, the compound (X) exerts no antiobesity or antidiabetic effect and the compound (XI) has only a weak transient anorectic effect which is of no practical value. In contrast, the compounds of this invention exert strong and long-lasting antiobesity and antidiabetic effects because of their having the particular substituent (halogen) at the particular (para) position of the benzene ring and having the particular secondary amine-type structure.

The starting compounds (V) and (VI) in Processes II and III can readily be synthesized from alanine according to the following reaction scheme. For example, an alanine derivative of formula (XII) is reduced and the resulting

aminoalcohol (XIII) is treated with a halogenating agent to give a starting compound (V') ($R^3$ = methyl in V) in the form of an acid addition salt, while the compound (XII) is partially reduced and the resulting compound (XIV) is then sulfonylated to give a compound (VI').

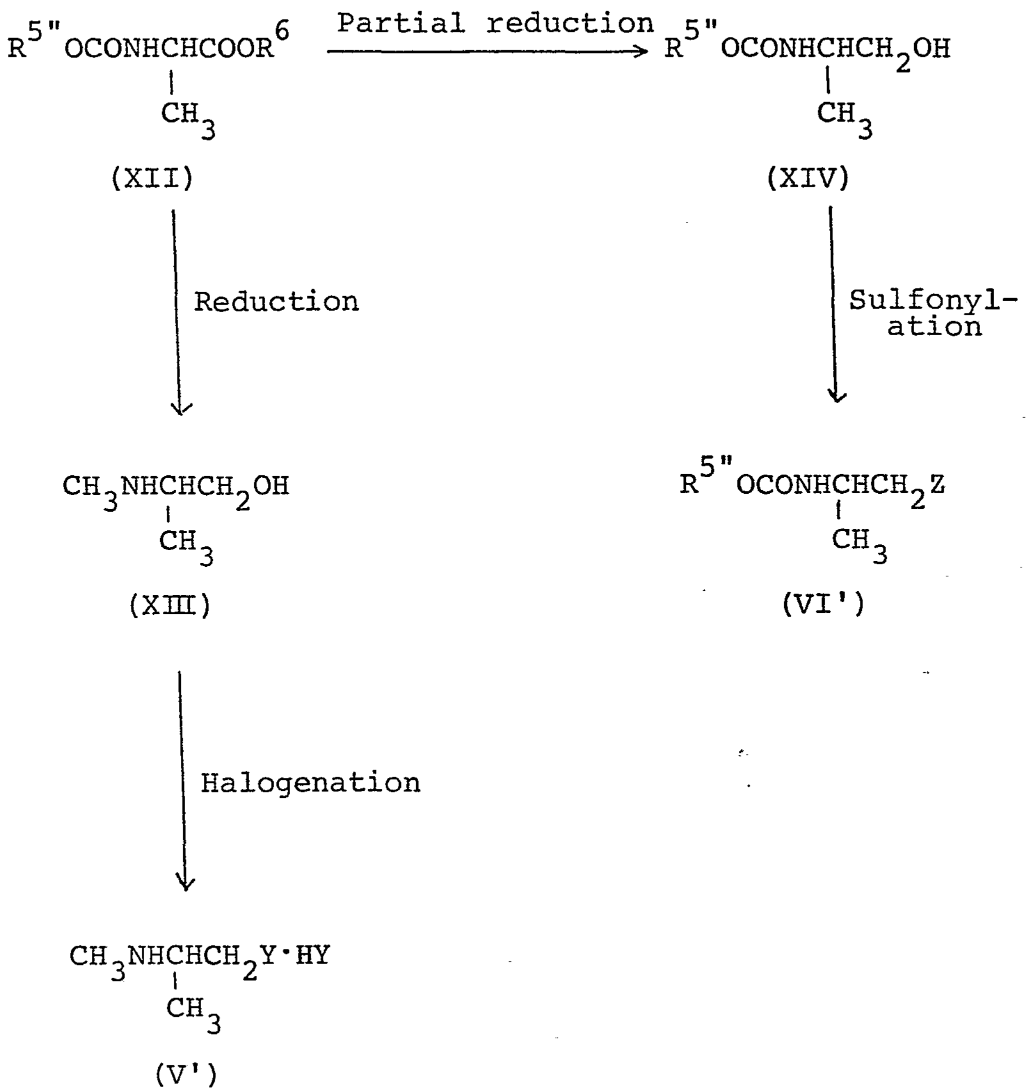


(XII) (XIV) (XIII) (VI') (V')

wherein $R^{5''}$ is as defined above, $R^6$ is hydrogen, lower alkyl or benzyl, Y is halogen, Z is an alkyl- or arylsulfonyloxy.

The reduction of a compound (XII) to synthesize the compound (XIII) proceeds advantageously by use of lithium

aluminum hydride and is usually carried out in a solvent such as ethyl ether, tetrahydrofuran, dimethoxyethane or the like, at a temperature in the range of from room temperature to the boiling point of the solvent. The halogenating agents which are used in halogenation of the resulting compound (XIII) include hydrobromic acid, thionyl chloride, phosphorus tribromide, etc. In the case where hydrobromic acid is used, its aqueous solution is used as it is. When thionyl chloride, phosphorus tribromide or the like is used, an anhydrous inert solvent, for example, chloroform, dichloromethane, benzene, toluene or the like may be used.

Other N-alkylalaninols may also be synthesized by reduction of an N-alkylalanine or its ester which is prepared in the manner described in Canadian Journal of Chemistry, Vol. 49, page 1968 (1971) or Vol. 51, page 1915 (1973), or in a similar manner, or by reduction of an N-acylalanine or its ester. Lithium aluminum hydride can be advantageously used also in these reduction reactions.

In the partial reduction of an alanine derivative (XII) to synthesize a compound (XIV), the reducing agent used is preferably sodium borohydride when the compound (XII) is an ester and diborane when (XII) is a free carboxylic acid. The reduction with sodium borohydride advantageously proceeds in a solvent such as methanol or ethanol at a temperature in the range of from room temperature to the boiling point of the solvent, while the reduction with diborane advantageously proceeds in a solvent such as tetrahydrofuran, dioxane, dimethoxyethane or diglyme at a temperature of 0 to 40°C to obtain the desired compound (XIV).

It is also possible to halogenate an alaninol (XV) which is a reduction product of alanine in the same way as in the reduction of (XIII) to form a compound (XVI), which is then acylated to give a compound (VI") as shown in the following reaction scheme and the thus obtained compound (VI") can

also be used as a starting material in the present invention

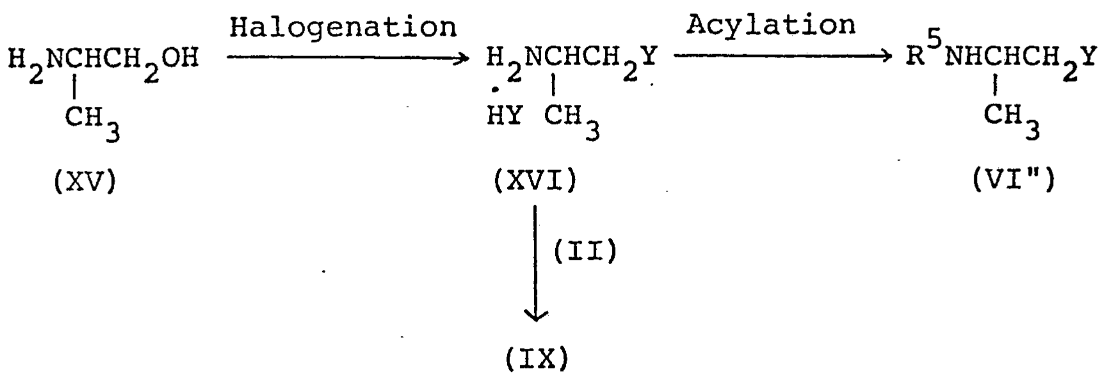


(XV) (XVI) (VI") (IX)

wherein $R^5$ and Y are as defined above.

Another starting compound (IX) for Process III can be synthesized in the manner described in South African Patent No. 67/3108 or in a manner similar thereto, or by reacting the thiophenol derivative (II) with the compound (XVI) in the presence of a base (e.g. sodium methoxide or sodium ethoxide) in an alcohol (e.g. methanol or ethanol) at from room temperature to the boiling point of the alcohol used.

In each case, the use of L-alanine or L-alaninol leads to the formation of the starting compound (V'), (VI'), (VI") or (IX) having the S-absolute configuration, and the use of D-alanine or D-alaninol to the formation of those having the R-absolute configuration, while the use of DL-alanine or DL-alaninol results in the formation of optically inactive compound (V'), (VI'), (VI") or (IX).

The following pharmacological experiments demonstrate the excellent utility of compounds (I) according to this invention.

A. Suppression of food intake in mice

Male ICR mice, 7 weeks old, were acclimatised to six-hours-a-day feeding (CE-2 powder, Clea Japan) for 7 days and those animals showing a constant food intake were used in groups of 6 individuals. Each test compound was dissolved in a 5% aqueous solution of gum arabic and orally

administered to mice at a dose level of 50 mg/kg as compound (I) at 0.5 hour before feeding on the 8th day. Control animals received a 5% solution of gum arabic only. The results are set forth in Table 1. Each figure in the table is the percent food intake with the food intake of control animals being taken as 100%.

Table 1

| Example No. of tested Compound (I) | Food intake (%) | |
|---|---|---|
| | 0.5 hour after administration | 1 hour after administration |
| 1 | 43.2 | 55.2 |
| 3 | 40.7 | 69.4 |
| 4 | 65.9 | 67.6 |
| 5 | 57.9 | 66.5 |
| 6 | 70.8 | 72.1 |
| 7 | 54.9 | 63.2 |
| The compound of Belgian Patent No. 653,101 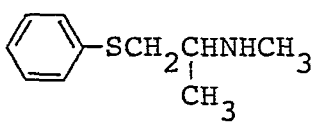 (acid fumarate) | 105.8(NS) | 111.8(NS) |
| The compound of South African Patent No. 67/3108 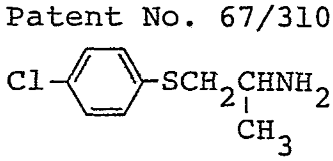 (hydrochloride) | 69.5 | 85.5(NS) |

In the table, the figures accompanied by NS in parentheses mean that the difference from control was not statistically significant. All other figures were significant at from $P<0.02$ to $P<0.001$.

B. Suppression of food intake in rats

Male 7-week-old rats of Sprague-Dawley strain were fasted for 24 hours and assigned to groups of 6 animals. A solution of 20 mg/kg of each test compound in distilled water was administered by oral gavage and after 0.5 hour, food (CE-2 powder) was given. The food intake in each group was measured at 1 hour and 2 hours thereafter and the results were compared with the food intake of control animals given distilled water only. The results are set forth in Table 2. The figures in the table denote the food intake percents, with the food intake of control animals being taken as 100%. By the term "gavage" is meant compulsory feeding.

Table 2

| Example No. of tested compound (I) | Food intake (%) | |
|---|---|---|
| | 1 hour after administration | 2 hours after administration |
| 1 | 43.9* | 61.9** |
| 8 | 38.8** | 56.1** |

Against control, ** $P<0.01$, * $P<0.05$

C. Effect on glucose tolerance in rats

(1) Male 7-week-old Sprague-Dawley rats and fatty rats (in groups of 5 animals) were fasted for 20 hours, and then, orally given 20 mg/kg or 50 mg/kg of 1-(4-chlorophenylthio)-2-methylaminopropane fumarate as dissolved in distilled water and after an interval of 30 minutes, 3 g/kg of glucose were orally administered. Immediately before the glucose loading and at 30, 60 and 120 minutes thereafter, blood samples were taken from the tail vein and the plasma levels of glucose were measured to

compute glucose areas. The results are shown in Table 3.

Table 3

| Rat | Dosage (mg/kg) | Glucose area (mg/100 ml·hr) |
|---|---|---|
| Sprague-Dawley | 0 (Control) | 151 |
| | 20 | 108* |
| | 50 | 35** |
| Fatty | 0 (Control) | 273 |
| | 20 | 198* |

Against each control (distilled water only),
* P<0.05, ** P<0.01

(2) Male 7-week-old rats of Sprague-Dawley strain (in groups of 5 animals) were fasted for 20 hours and orally given 20 mg/kg of each test compound as dissolved in distilled water. After 30 minutes, the animals were orally loaded with 3 g/kg of glucose. Blood samples were taken from tail veins and measured for glucose at 30 minutes when a peak glucose level would be reached. The results are set forth in Table 4.

Table 4

| Example No. of Tested Compound (I) | Blood sugar at 30 minutes after glucose loading (mg/100 ml) |
|---|---|
| 1 | 105** |
| 7 | 102** |
| Control (distilled water) | 146 |

Against control, * P<0.01, ** P<0.001

(3) Male 7-week-old rats of Sprague-Dawley strain (in groups of 5 animals) were fasted for 24 hours, and then, orally given test compounds as dissolved in distilled water. After a lapse of 30 minutes, 3 g/kg of glucose was orally administered and after another 30 minutes, blood samples were taken from tail veins and measured for glucose levels. Table 5 shows the results, with the glucose level of control animals being taken as 100%.

Table 5

| Example No. of Tested compound (I) | Dosage (mg/kg) | Blood sugar (%) |
|---|---|---|
| 1 | 20 | 53.2** |
| 8 | 1 | 86.3 |
| | 5 | 78.1* |
| | 10 | 45.9** |
| | 20 | 32.8** |

Against control, ** $P<0.001$, * $P<0.05$

D. Inhibition of insulin hypersecretion in rats

(1) Male 7-week-old Sprague-Dawley rats and fatty rats (in groups of 5 animals) were fasted for 20 hours and orally given 10, 20 or 50 mg/kg of 1-(4-chlorophenylthio)-2-methylaminopropane fumarate. After 30 minutes, 3 g/kg of glucose was orally administered to each animal. Immediately before glucose loading and at 30, 60 and 120 minutes thereafter, blood samples were collected from the tail vein and assayed for plasma insulin levels to compute insulin areas. The results are set forth in Table 6.

Table 6

| Rat | Dosage (mg/kg) | Insulin area (μU/ml·hr) |
|---|---|---|
| Sprague-Dawley | 0 (Control) | 73 |
| | 10 | 38* |
| | 20 | 33** |
| | 50 | 22*** |
| Fatty | 0 (Control) | 165 |
| | 20 | 33*** |

Against each control (distilled water only),
* $P<0.05$, ** $P<0.02$, *** $P<0.01$

(2) Male 7-week-old Sprague-Dawley rats (in groups of 5 animals) were fasted for 24 hours and, then, orally given 1, 5, 10 or 20 mg/kg of (S)-1-(4-chlorophenylthio)-2-methylaminopropane fumarate as dissolved in distilled water. After 30 minutes, 3 g/kg of glucose were orally administered to each animal. Then, after another 30 minutes, a blood sample was taken from the tail vein and the plasma was separated and assayed for insulin. The results were expressed as percents, with the value for distilled water control being taken as 100.

Table 7

| Dosage (mg/kg) | Plasma insulin (%) |
|---|---|
| 1 | 58.1* |
| 5 | 46.8* |
| 10 | 43.5** |
| 20 | 22.6** |

Against control, ** $P<0.001$, * $P<0.01$

E. Inhibition of fatty acid synthesis

Male 7-week-old Sprague-Dawley rats were acclimatised to 2-hours-a-day feeding (CE-2 powder, Clea Japan) for 10 days and assigned to groups of 5 animals. The animals were orally given 20 mg/kg of 1-(4-chlorophenylthio)-2-methylaminopropane fumarate as dissolved in distilled water, and,30 minutes thereafter, glucose-U-$^{14}$C (85 µCi/3 g/kg) was orally administered. After 60 minutes, each animal was sacrificed and the radioactivities incorporated into the fatty acid fractions of the liver and the epididymal adipose tissues were determined. The relative inhibition rates with respect to the control group given distilled water instead of the test compound were 46.6% in the liver and 58.8% in the adipose tissues.

F. Acute toxicity ($LD_{50}$)

Male 8-week-old mice of ICR strain (in groups of 8 animals) were orally given an aqueous solution of (S)-1-(4-chlorophenylthio)-2-methylaminopropane fumarate in doses of 500, 750, 1000, 1250 and 1500 mg/kg. The animals were fed and observed for 5 days and the $LD_{50}$ value was computed by probit analysis.

$$LD_{50} = 1070 \text{ mg/kg}$$

The following Reference Examples and Examples are further illustrative but by no means limitative of this invention.

Reference Example 1

In a mixture of ethanol (160 ml) and 2 N-NaOH (95 ml) was dissolved 4-chlorothiophenol (22.8 g), and chloroacetone (1.39 g) was added dropwise under ice-cooling and stirring. The mixture was further stirred under ice-cooling for 1.5 hours and 300 ml of water were added. After ice-cooling, the resultant crystalline precipitate was recovered by filtration to give 1-(4-chlorophenylthio)-2-propanone (30.6 g), m.p. 36-37°C.

Reference Example 2

In a mixture of ethanol (20 ml) and 2 N-NaOH (12 ml) was dissolved 4-fluorothiophenol (2.56 g), and chloroacetone (1.8 ml) was added dropwise under ice-cooling and stirring. The mixture was further stirred under ice-cooling for 2 hours, then diluted with water (50 ml), and extracted with ethyl ether. The ethyl ether layer was washed with water and dried over anhydrous magnesium sulfate, and the ethyl ether was distilled off to give 1-(4-fluorophenylthio)-2-propanone as an oil. Yield 3.7 g. The product was not purified but used directly in the subsequent process.

Similar procedures gave the following compounds:

1-(4-bromophenylthio)-2-propanone, m.p. 63-64°C

1-(3,4-dichlorophenylthio)-2-propanone, m.p. 36-37°C

Reference Example 3

(1) To a mixture of lithium aluminum hydride (28 g) and dry tetrahydrofuran (500 ml) was added dropwise a solution of N-carbobenzyloxy-L-alanine (55 g) in dry tetrahydrofuran under reflux. The mixture was further refluxed for 2 hours, and then ice-cooled, and acetone (60 ml) was added dropwise with stirring so as to decompose the excess lithium aluminum hydride. Then, water (100 ml) was added under ice-cooling and the mixture was stirred at room temperature for a while. The precipitate was filtered off and washed with ethyl ether and tetrahydrofuran. The filtrate was concentrated and the concentrate was dried over anhydrous potassium carbonate.

The solvent was distilled off and the residue was distilled under reduced pressure to give 18.45 g of (S)-2-methylaminopropanol, b.p. 67-80°C (11 mmHg).

Reduction of N-carboethoxy-L-alanine under the same conditions gave (S)-2-methylaminopropanol.

(2) In $CHCl_3$ (50 ml) was dissolved (S)-2-methylaminopropanol (10.4 g), followed by dropwise addition of thionyl chloride (10.2 ml) under ice-cooling, and the mixture was

refluxed for 2 hours. The solvent was distilled off and the crystalline residue was recrystallized from ethanol-isopropyl ether to give (S)-2-methylaminopropyl chloride hydrochloride (12.0 g). A portion of the product was purified by sublimation and subsequent recrystallization from ethanol-isopropyl ether gave colorless needles, m.p. 149-150.5°C, $[\alpha]_D^{22}$ + 2.9° (c = 2.0, in methanol).

Elemental analysis:

Calcd. for $C_4H_{10}ClN \cdot HCl$: C, 33.35; H, 7.70; N, 9.72
Found : C, 33.29; H, 7.70; N, 9.72

N-carbobenzyloxy-D-alanine as a starting compound was treated in the same manner as above [(1) and (2)] to give (R)-2-methylaminopropyl chloride hydrochloride, m.p. 149.5-151.5°C, $[\alpha]_D^{24}$ - 2.6° (c = 2.0, in methanol).

Reference Example 4

(1) To a mixture of sodium borohydride (1.36 g) and dry dioxane (20 ml) was added a solution of N-carbobenzyloxy-L-alanine (4.46 g) in dry dioxane (10 ml) dropwise under ice-cooling and stirring, followed by dropwise addition of a solution of boron trifluoride ethyl ether (6.2 ml) in dry dioxane (10 ml), and the mixture was stirred under ice-cooling for an hour. The mixture was acidified with water and acetic acid, the solvent was distilled off and the residue was extracted with ethyl acetate. The ethyl acetate layer was washed with water, aqueous sodium hydrogen carbonate and water in that order, and dried over anhydrous magnesium sulfate. The solvent was distilled off and the residue was crystallized from ice-cooled isopropyl ether to give (S)-2-benzyloxycarbonylaminopropanol (2.70 g). Recrystallization from isopropyl ether gave colorless needles, m.p. 77-78°C, $[\alpha]_D^{23}$ - 4.7° (c = 2.0, in methanol).

Elemental analysis:

Calcd. for $C_{11}H_{15}NO_3$: C, 63.14; H, 7.23; N, 6.69
Found : C, 63.08; H, 7.09; N, 6.54

(2) To a mixture of (S)-2-benzyloxycarbonylaminopropanol (2.09 g), dichloromethane (20 ml) and triethylamine (1.68 ml) was added mesyl chloride (0.92 ml) dropwise under cooling with ice-NaCl. The mixture was stirred under cooling for 40 minutes, after which it was washed with water and dried over anhydrous mgnesium sulfate. The solvent was then distilled off. The crystalline residue was recovered by filtration and washed with isopropyl ether to give (S)-N-benzyloxycarbonyl-(2-mesyloxy-1-methyl)ethylamine (2.4 g). Recrystallization from methanol gave colorless prisms, m.p. 104-105°C, $[\alpha]_D^{23}$ - 19.3° (c = 2.1, in methanol).

Elemental analysis:

| | | |
|---|---|---|
| Calcd. for $C_{12}H_{17}NO_5S$: | | C, 50.16; H, 5.96; N, 4.87 |
| Found | : | C, 50.04; H, 5.71; N, 4.59 |

Reference Example 5

(1) To a solution of N-ethoxycarbonyl-L-alanine methyl ester (18.9 g) in ethanol (100 ml) was added sodium borohydride (4.56 g), and the mixture was stirred at room temperature for 4 hours. Acetone was added thereto to decompose the excess reagent and the mixture was diluted with water until the precipitate dissolved. Then the mixture was made acidic with acetic acid and evaporated. A solution of the residue in water was saturated with sodium chloride and extracted with dichloromethane. The extract was dried over anhydrous magnesium sulfate and evaporated to yield (S)-2-ethoxycarbonylaminopropanol (13.4 g) as an oil.

(2) To a stirred mixture of the oil (13.4 g) obtained in (1), dichloromethane (150 ml) and triethylamine (14 ml), mesyl chloride (7.8 ml) was added dropwise with cooling with ice-salt. The mixture was stirred for 40 minutes, washed with water and dried over anhydrous magnesium sulfate. The solvent was evaporated off and the residue was crystallized from isopropyl ether to give (S)-N-ethoxycarbonyl-(2-mesyloxy-1-methyl)ethylamine as crystals (18.3 g). An

analytical sample was obtained by recrystallization from isopropanol as colorless needles, m.p. 63-64°C, $[\alpha]_D^{22}$ - 36.7° (c = 2, in methanol).

Elemental analysis:

Calcd. for $C_7H_{15}NO_5S$: C, 37.32; H, 6.71; N, 6.22

Found : C, 37.47; H, 6.82; N, 6.31

Reference Example 6

(1) A solution of L-alaninol (16 g) in 48% hydrobromic acid (80 ml) was heated on an oil bath with gradual distillation of the reaction mixture. During 4 hours, 45 ml of a distillate were obtained. Another 40 ml of 48% hydrobromic acid were added to the reaction mixture and heating was continued for 6 hours during which time another 20 ml of a distillate were obtained. The reaction mixture was concentrated to dryness under reduced pressure. To the residue were added acetone (50 ml) and benzene (50 ml) and the mixture was re-evaporated. Thus, (S)-2-aminopropyl bromide hydrobromide was obtained as a hygroscopic semisolid (35 g). This was used in the next reaction without further purification.

(2) To a stirred mixture of p-chlorothiophenol (14.5 g), methanol (80 ml), 28% sodium methoxide in methanol (45 ml), a solution of (S)-2-aminopropyl bromide hydrobromide (28.4 g) obtained in (1) in methanol (60 ml) was added dropwise. The mixture was stirred at room temperature for 2 hours and the solvent was evaporated. After dilution with water (500 ml), the mixture was made acidic with hydrochloric acid and washed with ethyl ether. Then the aqueous phase was made alkaline with aqueous sodium hydroxide and extracted with ethyl ether. The extract was washed with water, dried over anhydrous magnesium sulfate. Evaporation of the solvent followed by distillation under reduced pressure gave (S)-2-amino-1-(4-chlorophenylthio)-propane as an oil (14.6 g), b.p. 135-137°C (0.5 mmHg), $[\alpha]_D^{23}$ + 32.3° (c = 2, in methanol).

Elemental analysis:

Calcd. for $C_9H_{12}ClNS$: C, 53.59; H, 6.00; N, 6.94
Found : C, 53.59; H, 6.03; N, 7.06

Example 1

To a mixture of 1-(4-chlorophenylthio)-2-propanone (20 g), methylamine hydrochloride (13.5 g), a 40% methanolic solution of methylamine (62 g) and methanol (100 ml) was added $NaBH_3CN$ (4 g) with stirring, and the whole mixture was stirred at room temperature for 5 days. After addition of 3 N-NaOH (100 ml), the mixture was concentrated under reduced pressure and the concentrate was extracted with ethyl ether. The ethyl ether layer was washed with water and dried over anhydrous magnesium sulfate and the ethyl ether was distilled off. The residue was dissolved in methanol (20 ml), followed by addition of a solution of fumaric acid (6.5 g) in ethanol (80 ml). The solution was then diluted with ethyl ether to give crystals. Recrystallization from ethanol-isopropanol (3:1, v/v) gave 1-(4-chlorophenylthio)-2-methylaminopropane fumarate (11.2 g), m.p. 160-161°C.

Elemental analysis:

Calcd. for $C_{10}H_{14}ClNS \cdot 1/2C_4H_4O_4$:
C, 52.64; H, 5.89; N, 5.12
Found : C, 52.60; H, 5.85; N, 5.27

Example 2

A mixture of 1-(4-chlorophenylthio)-2-propanone (5.5 g), N-methylformamide (16.5 g) and formic acid (5.06 g) was heated on an oil bath at 145-150°C for 8 hours. After cooling, the mixture was diluted with 150 ml of water and extracted with ethyl ether. The ethyl ether layer was washed with water and the solvent was distilled off. To the residue was added 6 N-HCl (20 ml) and the mixture was heated under reflux for 2 hours. The reaction mixture was diluted with water (60 ml) and washed with ethyl ether to remove the solubles. The aqueous layer was made alkaline with 6 N-NaOH (30 ml) and extracted with ethyl ether. The ethyl ether layer was washed with water and dried over

anhydrous magnesium sulfate, and the ethyl ether was distilled off. The oily residue was distilled under reduced pressure to give the free base of 1-(4-chlorophenylthio)-2-methylaminopropane (3.82 g), b.p. 103-104°C (0.2 mm Hg).

Elemental analysis:

Calcd. for $C_{10}H_{14}ClNS$: C, 55.67; H, 6.54; N, 6.49
Found : C, 55.43; H, 6.66; N, 6.42

In ethyl ether (10 ml) were dissolved 2 g of this free base, and an excess of methanolic hydrochloric acid was added. The mixture was diluted with ethyl ether. The resultant crystalline precipitate was recovered by filtration and recrystallized from ethanol-ethyl ether to give 1-(4-chlorophenylthio)-2-methylaminopropane hydrochloride (2.04), m.p. 127-128°C.

Elemental analysis:

Calcd. for $C_{10}H_{14}ClNS \cdot HCl$: C, 47.62; H, 6.00; N, 5.55
Found : C, 47.72; H, 5.97; N, 5.56

To a hot solution of fumaric acid (0.27 g) in isopropanol (4 ml) was added 1 g of the above free base, and the mixture was allowed to cool to give 1-(4-chlorophenylthio)-2-methylaminopropane fumarate (1.18 g), m.p. 160-161°C. In infrared absorption spectrum, this product was in complete agreement with the compound obtained in Example 1.

Example 3

In a 3 M solution (20 ml) of ethylamine in methanol was dissolved 1-(4-chlorophenylthio)-2-propanone (2 g), followed by addition of 15% methanolic HCl (4 ml) and $NaBH_3CN$ (0.4 g). The whole mixture was stirred at room temperature for 4 days. After addition of 3 N-NaOH (10 ml), the mixture was concentrated under reduced pressure and the concentrate was extracted with ethyl ether. The ethyl ether layer was washed with water, followed by drying over anhydrous magnesium sulfate, and the ethyl ether was distilled off. The residue was dissolved in ethanol (5 ml),

and a solution of fumaric acid (0.8 g) in ethanol (12 ml) was added. After concentration, acetone was added. The resulting crystals were recrystallized from ethanol to give 1-(4-chlorophenylthio)-2-ethylaminopropane fumarate as crystals melting at 150-153°C. Yield 1.0 g.

Elemental analysis:

Calcd. for $C_{11}H_{16}ClNS \cdot 1/2C_4H_4O_4$:
C, 54.25; H, 6.30; N, 4.87

Found : C, 54.00; H, 6.39; N, 5.02

Example 4

The reaction procedure of Example 3 was repeated except that a solution of monoethanolamine (3.7 g) in methanol (20 ml) was used in lieu of ethylamine in ethanol. The procedure gave crystals of 1-(4-chlorophenylthio)-2-(2-hydroxyethylamino)propane fumarate, melting 138-139°C. Yield 1.25 g.

Elemental analysis:

Calcd. for $C_{11}H_{16}ClNOS \cdot 1/2C_4H_4O_4$:
C, 51.40; H, 5.97; N, 4.61

Found : C, 51.26; H, 5.91; N, 4.82

Example 5

To a mixture of 1-(4-fluorophenylthio)-2-propanone (3.7 g), a 40% methanolic solution of methylamine (16 g), methanol (20 ml), and 15% methanolic HCl (8 ml) was added $NaBH_3CN$ (0.8 g), and the whole mixture was stirred at room temperature for 4 days. After addition of 3 N-NaOH (20 ml), the mixture was concentrated under reduced pressure and extracted with ethyl ether. The ethyl ether layer was washed with water and dried over anhydrous magnesium sulfate, and the solvent was distilled off. To the residue was added a solution of fumaric acid (1.7 g) in ethanol (25 ml), and the small amounts of insolubles were filtered off. The ethanol was then distilled off. Ethyl ether was added to the residue and the resulting crystals were recrystallized from ethanol-ethyl ether to give an acid fumarate of 1-(4-fluorophenylthio)-2-methylaminopropane,

melting at 123-124°C. Yield 2.28 g.

Elemental analysis:

Calcd. for $C_{10}H_{14}FNS \cdot C_4H_4O_4$:
C, 53.32; H, 5.75; N, 4.44

Found : C, 53.12; H, 5.68; N, 4.65

Example 6

To a mixture of 1-(4-bromophenylthio)-2-propanone (2.45 g), methylamine hydrochloride (1.35 g), a 40% methanolic solution of methylamine (6.2 g), and methanol (10 ml) was added $NaBH_3CN$ (0.4 g), and the whole mixture was stirred at room temperature for 4 days. After addition of 3 N-NaOH (10 ml), the mixture was concentrated under reduced pressure and extracted with ethyl ether. The ethyl ether layer was washed with water and dried over anhydrous magnesium sulfate, and the ethyl ether was distilled off. To the residue was added a solution of fumaric acid (1.2 g) in ethanol (18 ml),and the small amounts of insolubles were filtered off. The filtrate was concentrated, and ethyl ether was added. The resulting crystals were recrystallized from ethanol-ethyl ether to give 1.47 g of an acid fumarate of 1-(4-bromophenylthio)-2-methylamino-propane, m.p. 126-128°C.

Elemental analysis:

Calcd. for $C_{10}H_{14}BrNS \cdot C_4H_4O_4$:
C, 44.69; H, 4.82; N, 3.72

Found : C, 44.76; H, 4.86; N, 3.86

Example 7

To a mixture of 1-(3,4-dichlorophenylthio)-2-propanone (1.88 g), methylamine hydrochloride (1.35 g), a 40% methanolic solution of methylamine (6.2 g) and methanol (10 ml) was added $NaBH_3CN$ (0.4 g), and the whole mixture was stirred at room temperature for 7 days. After addition of 4 N-NaOH (10 ml), the mixture was concentrated under reduced pressure and extracted with chloroform. The chloroform layer was washed with water and dried over anhydrous magnesium sulfate, and the chloroform was distilled

off. To the residue was added a solution of fumaric acid (0.7 g) in ethanol (10 ml) and the mixture was cooled. The resultant crystalline precipitate was recovered by filtration and recrystallized from ethanol to give an acid fumarate of 1-(3,4-dichlorophenylthio)-2-methylamino-propane (1.05 g), m.p. 143-145°C.

Elemental analysis:

Calcd. for $C_{10}H_{13}Cl_2NS \cdot C_4H_4O_4$:
C, 45.91; H, 4.68; N, 3.82

Found : C, 45.89; H, 4.65; N, 4.00

Example 8

To a mixture of p-chlorothiophenol (11.5 g), methanol (80 ml) and a 28% methanolic solution of sodium methoxide (32 ml) was added dropwise a solution of (S)-2-methylaminopropyl chloride hydrochloride (11.5 g) in methanol (80 ml) under reflux during 1 hour and 20 minutes. The mixture was further refluxed for 2 hours. After addition of water (50 ml) and removal of the methanol by distillation, the mixture was acidified with 6 N-HCl and washed with ethyl ether. The aqueous layer was made alkaline with 6 N-NaOH and extracted with ethyl ether. The extract was washed with 1 N-NaOH and aqueous sodium chloride in that order and dried over anhydrous potassium carbonate. The solvent was then distilled off and the oily residue was distilled under reduced pressure to give (S)-1-(4-chlorophenylthio)-2-methylaminopropane (15.3 g), b.p. 107-108°C (0.6 mm Hg).

Fumaric acid (4.17 g) was added to ethanol (20 ml) and the above oil was added dropwise under heating. After additional heating for a while, isopropyl ether was added and the mixture was allowed to cool. The resultant crystalline precipitate was recovered by filtration and recrystallized from ethanol-isopropyl ether to give (S)-1-(4-chlorophenylthio)-2-methylaminopropane fumarate (16.2 g), m.p. 140.5-142°C, $[\alpha]_D^{25}$ + 12.2° (c = 2.0, in methanol).

Elemental analysis:

Calcd. for $C_{10}H_{14}ClNS \cdot 1/2C_4H_4O_4$:

C, 52.64; H, 5.89; N, 5.12

Found : C, 52.86; H, 5.84; N, 5.39

Example 9

As in Example 8, (S)-2-methylaminopropyl chloride hydrochloride was reacted with p-fluorothiophenol and the reaction product was converted to a salt with fumaric acid. The above procedure gave an acid fumarate of (S)-1-(4-fluorophenylthio)-2-methylaminopropane melting at 127-128°C, yield 94.4%. $[\alpha]_D^{23}$ + 8.6° (c = 2.0, in methanol)

Elemental analysis:

Calcd. for $C_{10}H_{14}FNS \cdot C_4H_4O_4$:

C, 53.32; H, 5.75; N, 4.44

Found : C, 53.16; H, 5.82; N, 4.51

Example 10

As in Example 8, (S)-2-methylaminopropyl chloride hydrochloride was reacted with 3,4-dichlorothiophenol and the reaction product was converted to a salt with fumaric acid. The above procedure gave an acid fumarate of (S)-1-(3,4-dichlorophenylthio)-2-methylaminopropane melting at 120-121°C in 68.3% yield. $[\alpha]_D^{23}$ + 9.1° (c = 2.0, in methanol)

Elemental analysis:

Calcd. for $C_{10}H_{13}NCl_2S \cdot C_4H_4O_4$:

C, 45.91; H, 4.68; N, 3.82

Found : C, 45.92; H, 4.68; N, 3.99

Example 11

As in Example 8, (R)-2-methylaminopropyl chloride hydrochloride was reacted with p-chlorothiophenol and the reaction product was converted to a salt with fumaric acid. The above procedure gave (R)-1-(4-chlorophenylthio)-2-methylaminopropane fumarate melting at 140.5-141.5° in 74% yield. $[\alpha]_D^{25}$ - 12.3° (c = 2.0, in methanol)

Elemental analysis:

Calcd. for $C_{10}H_{14}ClNS \cdot 1/2C_4H_4O_4$:

C, 52.64; H, 5.89; N, 5.12

Found : C, 52.73; H, 5.72; N, 5.09

Example 12

(1) A mixture of p-chlorothiophenol (0.72 g), (S)-N-benzyloxycarbonyl-(2-mesyloxy-1-methyl)ethylamine (1.44 g), sodium iodide (0.2 g), methanol (15 ml) and a 28% methanolic solution of sodium methoxide (1.0 g) was stirred at room temperature for 4 hours and under reflux for 2 hours. Then, the methanol was distilled off. Water was added to the residue and extraction was carried out with ethyl ether. The ethyl ether layer was washed with aqueous sodium hydroxide solution and water, and was dried over anhydrous magnesium sulfate. The solvent was distilled off and hexane was added to the residue to give (S)-2-benzyloxycarbonylamino-1-(4-chlorophenylthio)propane as crystals (1.30 g). Recrystallization from isopropyl ether gave colorless prisms, m.p. 66-67°C, $[\alpha]_D^{23}$ + 53.7° (c = 2.0, in methanol).

Elemental analysis:

Calcd. for $C_{17}H_{18}ClNO_2S$: C, 60.80; H, 5.40; N, 4.17

Found : C, 60.81; H, 5.24; N, 4.09

(2) To a solution of (S)-2-benzyloxycarbonylamino-1-(4-chlorophenylthio)propane (1.0 g) in N,N-dimethylformamide (6 ml) was added sodium hydride (60% oil dispersion, 0.12 g) with stirring and the mixture was further stirred for 40 minutes. The mixture was cooled with ice and methyl iodide (0.2 ml) was added dropwise. The mixture was stirred under ice-cooling for 15 minutes and at room temperature for 30 minutes. Then water was added and extraction was carried out with ethyl acetate. The organic layer was washed with water and dried over anhydrous magnesium sulfate. Removal of the solvent by distillation gave an oil (0.90 g) of (S)-2-(N-benzyloxycarbonyl)methylamino-1-(4-chlorophenylthio)propane.

(3) To a mixture of (S)-2-(N-benzyloxycarbonyl)-methylamino-1-(4-chlorophenylthio)propane (0.7 g), acetic acid (1 ml) and anisole (1 ml) was added 25% hydrobromic acid/acetic acid (3.5 ml) dropwise under ice-cooling and

stirring. After stirring for 2.5 hours, water was added and extraction was carried out with ethyl ether. The organic layer was washed with water and dried over anhydrous magnesium sulfate. Removal of the solvent by distillation gave an oil (0.4 g) of (S)-1-(4-chlorophenylthio)-2-methylaminopropane. This oil was dissolved in ethanol (5 ml), followed by addition of a solution of fumaric acid (0.12 g) in ethanol (3 ml) and, then, isopropyl ether was added. The resultant crystalline precipitate was recovered by filtration to give (S)-1-(4-chlorophenylthio)-2-methylaminopropane fumarate (0.50 g). Recrystallization from isopropanol-ethyl ether gave colorless crystals, m.p. 138-139°C, $[\alpha]_D^{23}$ + 12.4° (c = 2.0, in methanol). In IR and NMR spectra, this product was in complete agreement with the compound obtained in Example 8.

<u>Example 13</u>

(1) To a solution of sodium metal (0.46 g) in ethanol (40 ml), were added p-chlorothiophenol (2.89 g), (S)-N-ethoxycarbonyl-(2-mesyloxy-1-methyl)ethylamine (4.5 g) and sodium iodide (0.3 g). The mixture was stirred at room temperature for 2 hours and diluted with water to give (S)-1-(4-chlorophenylthio)-2-ethoxycarbonylaminopropane as crystals (4.1 g). Recrystallization from isopropyl ether gave colorless needles, m.p. 69-70°C, $[\alpha]_D^{22}$ + 37.1° (c = 2, in methanol).

Elemental analysis:

Calcd. for $C_{12}H_{16}ClNO_2S$: C, 52.64; H, 5.89; N, 5.12

Found : C, 52.87; H, 5.73; N, 5.14

(2) To a stirred, ice-cooled mixture of (S)-2-amino-1-(4-chlorophenylthio)propane (1.01 g), dichloromethane (6 ml), sodium carbonate (0.6 g) and water (6 ml), was added dropwise ethyl chloroformate (0.49 ml). After the mixture was stirred with cooling for 30 minutes, the dichloromethane layer was separated, washed successively with diluted hydrochloric acid, aqueous sodium bicarbonate and water, and dried over anhydrous magnesium sulfate.

Evaporation of the solvent followed by addition of hexane gave (S)-1-(4-chlorophenylthio)-2-ethoxycarbonylamino-propane as crystals (1.08 g), m.p. 70-70.5°C, $[\alpha]_D^{23}$ + 36.9° (c = 1, in methanol). This product was identical with the compound obtained in (1) above in IR and NMR spectra.

(3) To a stirred solution of (S)-1-(4-chlorophenyl-thio)-2-ethoxycarbonylaminopropane (2.74 g) in N,N-dimethylformamide (20 ml), sodium hydride (60% in oil, 0.44 g) was added portionwise. The mixture was stirred for 30 minutes and cooled with ice. Methyl iodide (0.68 ml) was added dropwise thereto and the mixture was stirred for 3 hours with ice-cooling and for 1 hour at room temperature. The reaction mixture was poured into ice-water (150 ml) and extracted with ethyl ether. The extract was washed with water and dried over anhydrous magnesium sulfate. Evaporation of the solvent gave (S)-1-(4-chlorophenylthio)-2-(N-ethoxycarbonyl)methylaminopropane as an oil.

(4) To the oil obtained in (3) was added 48% hydrobromic acid (8 ml) and the mixture was heated under reflux for 1 hour. The hydrobromic acid was evaporated off under reduced pressure. The residue was dissolved in water and washed with ethyl ether. The aqueous phase was made alkaline with solid sodium bicarbonate followed by aqueous sodium hydroxide, and extracted with ethyl ether. The extract was washed with water and dried over anhydrous magnesium sulfate. Evaporation of the solvent gave (S)-1-(4-chlorophenylthio)-2-methylaminopropan as an oil (1.71 g). A hot solution of fumaric acid (0.46 g) in ethanol (8 ml) was added to the oil. The solution was concentrated and diluted with isopropyl ether to yield (S)-1-(4-chloro-phenylthio)-2-methylaminopropane fumarate (2.03 g). Recrystallization from isopropanol gave colorless crystals (1.86 g), m.p. 140-142°C, $[\alpha]_D^{23}$ + 12.2° (c = 2, in methanol). This product was identical with the compound obtained in Example 8 in IR and NMR spectra.

Example 14

(1) To a solution of (S)-2-amino-1-(4-chlorophenylthio)propane (2.0 g ) in pyridine (8 ml), was added dropwise acetic anhydride (3 ml) under ice-cooling. The mixture was allowed to stand at room temperature for 1 hour, diluted with ice-water and extracted with ethyl ether. The extract was washed successively with diluted sulfuric acid, aqueous sodium bicarbonate and water, and dried over anhydrous magnesium sulfate. Evaporation of the solvent followed by addition of hexane gave (S)-2-acetamido-1-(4-chlorophenylthio)propane as crystals (2.18 g). Recrystallization from isopropyl ether gave colorless prisms, m.p. 88-89°C, $[\alpha]_D^{23}$ + 17.6° (c = 2, in methanol).

Elemental analysis:

Calcd. for $C_{11}H_{14}ClNOS$: C, 54.20; H, 5.79; N, 5.75
Found : C, 54.23; H, 5.74; N, 5.71

(2) To a stirred solution of (S)-2-acetamido-1-(4-chlorophenylthio)propane (2.44 g) in N,N-dimethylformamide (20 ml), was added portionwise sodium hydride (60% in oil, 0.44 g). The mixture was stirred at room temperature for 1 hour. Then methyl iodide (0.68 ml) was added dropwise thereto under ice-cooling. The mixture was stirred under cooling for 2 hours, poured into ice-water (200 ml), and extracted with ethyl ether. The extract was washed with water and dried over anhydrous magnesium sulfate. Evaporation of the solvent gave a crude oil of (S)-2-(N-methylacetamido)-1-(4-chlorophenylthio)propane. To the oil was added concentrated hydrochloric acid (15 ml) and the mixture was refluxed with stirring for 28 hours. After dilution with water, the mixture was washed with ethyl ether. The aqueous phase was made alkaline with sodium hydroxide and extracted with ethyl ether. The extract was washed with water, and dried over anhydrous magnesium sulfate. Evaporation of the solvent left an oil (1.09 g) which was dissolved in a solution of fumaric acid

(293 mg) in hot ethanol (6 ml). The solution was concentrated and diluted with isopropyl ether to yield (S)-1-(4-chlorophenylthio)-2-methylaminopropane fumarate as crystals (1.39 g). Recrystallization from isopropanol gave colorless crystals (1.27 g), m.p. 140.5-142°C, $[\alpha]_D^{23}$ + 12.1° (c = 2, in methanol). This product was identical with the compound obtained in Example 8 in IR and NMR spectra.

Example 15

When the compound (I) of this invention is used as a therapeutic agent for obesity or as a prophylactic and therapeutic agent for diabetes, it can be employed for example in the following formulations and dosage forms.

A. Tablets

| | |
|---|---|
| (1) 1-(4-chlorophenylthio)-2-methylaminopropane fumarate | 10 g |
| (2) Lactose | 90 g |
| (3) Corn starch | 29 g |
| (4) Magnesium stearate | 1 g |
| | 130 g/1000 tablets |

The entire amounts of (1) and (2) are mixed with 17 g of corn starch, and the mixture is granulated with the aid of a paste prepared from another 7 g portion of corn starch. To the granules are added the remaining 5 g of corn starch and the entire amount of (4). The whole mixture is compressed on a compression molding machine to give 1000 tablets, 7 mm in diameter, each containing 10 mg of (1).

B. Capsules

| | |
|---|---|
| (1) (S)-1-(4-chlorophenylthio)-2-methylaminopropane fumarate | 10 g |
| (2) Lactose | 135 g |
| (3) Finely divided cellulose | 70 g |
| (4) Magnesium stearate | 5 g |
| | 220 g per 1000 capsules |

The entire amounts of the above components are admixed and filled into 1000 gelatin capsules (No.3, Japanese Pharmacopeia, 9th Revised Edition) to give capsules each

containing 10 mg of (1).

C. Injection

| | |
|---|---|
| (1) 1-(4-chlorophenylthio)-2-methylaminopropane fumarate | 1 g |
| (2) sodium chloride | 9 g |

The whole amounts of the above components are dissolved in 1000 ml of distilled water and the solution is distributed into 1000 amber-colored ampoules at the rate of 1 ml per ampoule, followed by purging with $N_2$ and sealing. The entire process is carried out under sterile conditions.

By the symbol "P" referred to above in connection with "difference from control" is meant a significant difference against control according to the Student's T Test described, e.g., in Snedecor G.W. et al : "Statistical Methods, 6th Edition (1967), Published by Iowa State University Press.

For example, "P=0.02" means that the probability of error is 2 percent.

CLAIMS

1. A compound of the formula:

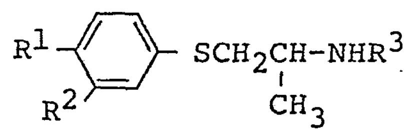

wherein $R^1$ is halogen,
$R^2$ is hydrogen or halogen, and
$R^3$ is methyl, ethyl or hydroxyethyl,
or an acid addition salt thereof.

2. A compound according to claim 1, wherein $R^3$ is methyl.

3. A compound according to claim 1, wherein $R^1$ is chlorine.

4. A compound according to claim 1, wherein the acid addition salt is a physiologically acceptable salt.

5. A compound according to claim 4, wherein the physiologically acceptable salt is a salt with fumaric acid.

6. A compound according to claim 1, which is in the form of a mixture of an isomer having S-configuration and an isomer having R-configuration.

7. A compound according to claim 1, which is in the form of an isomer having S-configuration.

8. A compound according to claim 1, which is 1-(3,4-dichlorophenylthio)-2-methylaminopropane.

9. A compound according to claim 1, which is (S)-1-(3,4-dichlorophenylthio)-2-methylaminopropane.

10. A compound according to claim 1, which is 1-(4-chlorophenylthio)-2-methylaminopropane.

11. A compound according to claim 1, which is (S)-1-(4-chlorophenylthio)-2-methylaminopropane.

12. A compound according to claim 1, which is (S)-1-(4-chlorophenylthio)-2-methylaminopropane fumarate.

13. A pharmaceutical composition which comprises, as an active ingredient, an effective amount of a compound

as claimed in any one of claims 1 to 12 together with a pharmaceutically acceptable carrier, vehicle or diluent therefor.

14. A compound as claimed in any one of claims 1 to 12 or a composition as claimed in claim 13 for use as a medicament.

15. A method for producing a compound of the formula (I):

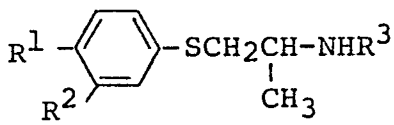

(I)

wherein $R^1$ is halogen,

$R^2$ is hydrogen or halogen, and

$R^3$ is methyl, ethyl or hydroxyethyl,

or an acid addition salt thereof,

which comprises

a) subjecting a compound of the formula:

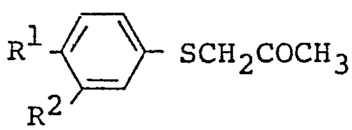

wherein $R^1$ and $R^2$ are as defined above,

and a compound of the formula:

$$R^3NH_2$$

wherein $R^3$ is as defined,

to condensation under reductive conditions, or

b) hydrolyzing a compound of the formula:

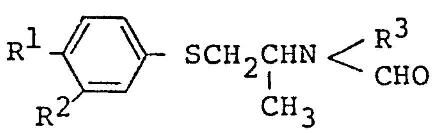

wherein all the symbols are as defined above, or

c) reacting a compound of the formula:

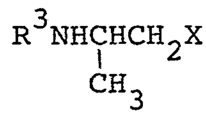

wherein X is a leaving group and $R^3$ is as defined above, with a compound of the formula:

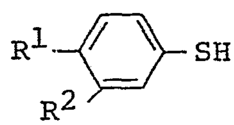

wherein $R^1$ and $R^2$ are as defined above, or

d) deacylating a compound of the formula:

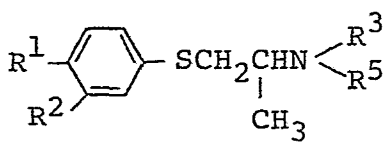

wherein $R^5$ is acyl and all other symbols are as defined above, and

e) if desired, converting the thus obtained compound of the formula (I) into an acid addition salt thereof.

0053015

European Patent Office

# EUROPEAN SEARCH REPORT

Application number
EP 81305485.5

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.3) |
|---|---|---|---|
| | US - A - 2 774 770 (JAMES F. KERWIN)<br>* Claim 1; column 2 *<br>-- | 1,15 | C 07 C 149/42<br>C 07 C 148/00<br>A 61 K 31/135 |
| | GB - A - 1 155 549 (CIBA)<br>* Claims 1,8,10 *<br>-- | 1,13,<br>14,15 | |
| D,X | BE - A1 - 653 101 (LABORATOIRES S.M.B.)<br>* Claims 1,2,17,18,39 *<br>---- | 1,13,<br>14,15 | |

TECHNICAL FIELDS SEARCHED (Int. Cl.3)

C 07 C 149/00
C 07 C 148/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 15-02-1982 | REIF |